# EUROPÄISCHE PATENTANMELDUNG

(11) **EP 3 861 951 A1**
(43) Veröffentlichungstag der Anmeldung: **11.08.2021**
(21) Anmeldenummer: 21151234.8
(22) Anmeldetag: 12.01.2021
(51) Int. Cl.: A61B 18/12, A61B 90/00, A61B 18/14, A61B 18/00, A61B 17/32

(54) **ELEKTROCHIRURGISCHES SYSTEM, ELEKTROCHIRURGISCHES STEUERGERÄT, ELEKTROCHIRURGISCHES INSTRUMENT, UND VERFAHREN ZUM BETRIEB EINES ELEKTROCHIRURGISCHEN SYSTEMS**

(30) Priorität: 10.02.2020 DE 102020103307
(71) Anmelder: OLYMPUS WINTER & IBE GMBH, 22045 Hamburg (DE)
(72) Erfinder: Kwik, Anne, 14165 Berlin (DE); Bombor, Dirk, 15711 Königs Wusterhausen (DE)
(74) Vertreter: Noack, Andreas

(57) **Zusammenfassung**

Es wird ein elektrochirurgisches System vorgestellt, umfassend: ein elektrochirurgisches Steuergerät (10), wenigstens ein elektrochirurgisches Instrument (11, 12), welches mit dem elektrochirurgischen Steuergerät (10) verbindbar ist, wobei das wenigstens eine elektrochirurgische Instrument (11, 12) ein erstes Speicherelement (30, 31) umfasst, auf welchem das elektrochirurgische Instrument (11, 12) charakterisierende Daten hinterlegt oder hinterlegbar sind, das wenigstens eine elektrochirurgische Instrument (11, 12) eingerichtet ist, nach Verbindung mit dem elektrochirurgischen Steuergerät (10) die das elektrochirurgische Instrument (11, 12) charakterisierenden Daten zumindest teilweise als erste Daten an das elektrochirurgische Steuergerät (10) zu übermitteln, und das elektrochirurgische Steuergerät (10) eingerichtet ist, auf Basis der von dem elektrochirurgischen Instrument (11, 12) übermittelten ersten Daten zulässige Betriebsparameter des elektrochirurgischen Instruments (11, 12) festzulegen.

Das elektrochirurgische System zeichnet sich dadurch aus, dass auf dem ersten Speicherelement (30, 31) oder einem zweiten Speicherelement (40, 41) des elektrochirurgischen Instruments (11, 12) das elektrochirurgische Instrument (11, 12) authentifizierende Daten hinterlegt oder hinterlegbar sind, das elektrochirurgische Steuergerät (10) eingerichtet ist, nach Verbindung mit dem elektrochirurgischen Instrument (11, 12) zweite Daten an das elektrochirurgische Instrument (11, 12) zu übermitteln, das wenigstens eine elektrochirurgische Instrument (11, 12) eingerichtet ist, aus den von dem elektrochirurgischen Steuergerät (10) übermittelten zweiten Daten und den das elektrochirurgische Instrument (11, 12) authentifizierenden Daten abgeleitete dritte Daten an das elektrochirurgische Steuergerät (10) zu übermitteln, und das elektrochirurgische Steuergerät (10) eingerichtet ist, anhand der von dem elektrochirurgischen Instrument (11, 12) übermittelten dritten Daten einen zulässigen Funktionsumfang für das elektrochirurgische Instrument (11, 12) festzulegen.

## Beschreibung

Die Erfindung betrifft ein Elektrochirurgisches System, umfassend ein elektrochirurgisches Steuergerät und wenigstens ein elektrochirurgisches Instrument, welches mit dem elektrochirurgischen Steuergerät verbindbar ist, wobei: das wenigstens eine elektrochirurgische Instrument ein erstes Speicherelement umfasst, auf welchem das elektrochirurgische Instrument charakterisierende Daten hinterlegt oder hinterlegbar sind; das wenigstens eine elektrochirurgische Instrument eingerichtet ist, nach Verbindung mit dem elektrochirurgischen Steuergerät die das elektrochirurgische Instrument charakterisierenden Daten zumindest teilweise als erste Daten an das elektrochirurgische Steuergerät zu übermitteln; und das elektrochirurgische Steuergerät eingerichtet ist, auf Basis der von dem elektrochirurgischen Instrument übermittelten ersten Daten zulässige Betriebsparameter des elektrochirurgischen Instruments festzulegen.

Weiterhin betrifft die Erfindung ein elektrochirurgisches Steuergerät, ein elektrochirurgisches Instrument, und ein Verfahren zum Betrieb eines elektrochirurgischen Systems.

Elektrochirurgische Systeme werden in der Medizin eingesetzt, um Gewebe eines menschlichen oder tierischen Patienten zu behandeln. Dabei wird zumeist in dem elektrochirurgischen Steuergerät ein hochfrequenter Wechselstrom erzeugt, welcher an das elektrochirurgische Instrument geleitet wird, wo er mittels einer oder mehrerer Behandlungselektroden in das Gewebe eingeleitet wird. In anderen Verfahren wird durch den Wechselstrom in einem Fluid ein Plasma erzeugt, welches dann mit dem Gewebe in Kontakt gebracht wird.

In manchen elektrochirurgischen Systemen wird der Wechselstrom genutzt, um in dem elektrochirurgischen Instrument eine Ultraschallschwingung anzuregen, die dann über eine Sonotrode in das zu behandelnde Gewebe eingebracht wird.

Vereinzelt wird in elektrochirurgischen Systemen auch mit Gleichstrom gearbeitet.

Das elektrochirurgische Steuergerät kann beispielsweise ein elektrochirurgischer Generator oder ein Ultraschallgenerator sein.

Im Zuge der technischen Entwicklung wurden zahlreiche neuartige elektrochirurgische Instrumente entwickelt, welche für bestimmte chirurgische Prozeduren besonders geeignet sind. Für solche neuartigen Instrumente wurden oftmals besondere Wellenformen für den hochfrequenten Wechselstrom definiert, um diese besonders wirksam einsetzen zu können.

Um möglichst viele unterschiedliche elektrochirurgische Instrumente ansteuern zu können, sind moderne elektrochirurgische Steuergeräte daher in der Lage, eine entsprechend hohe Anzahl an Wellenformen bei unterschiedlichen elektrischen Leistungen bereitzustellen.

Es liegt auf der Hand, dass dabei nicht jede Wellenform mit jedem Instrument kompatibel ist. Für einen sicheren Betrieb eines elektrochirurgischen Systems ist es daher erforderlich, dass das Steuergerät nur solche Wellenformen und Leistungen zur Verfügung stellt, die das jeweils angeschlossene Instrument auch verarbeiten kann.

Dazu ist es bekannt, das elektrochirurgische Instrument mit einem Speicher auszustatten, auf welchem das elektrochirurgische Instrument charakterisierende Daten hinterlegt sind. Diese Daten können bei Verbindung des Instruments mit dem Steuergerät an das Steuergerät übertragen werden, und das Steuergerät kann anhand dieser Daten zulässige Betriebsparameter des elektrochirurgischen Instruments festlegen.

Bei den das elektrochirurgische Instrument charakterisierenden Daten kann es sich um einen einfachen Identifizierungscode handeln, beispielsweise eine Typkennzeichnung. Die charakterisierenden Daten können auch direkt zulässige Betriebsparameter enthalten, wie z.B. erlaubte Spannungen und/oder Leistungen eines von dem Steuergerät abzugebenden Wechselstroms.

Nach dem Stand der Technik ausgeführte elektrochirurgische Systeme können eine zuverlässige und sichere Funktion bereitstellen, wenn sichergestellt ist, dass die auf dem Speicher des elektrochirurgischen Instruments hinterlegten Daten das entsprechende elektrochirurgische Instrument zutreffend charakterisieren. Dies wird durch Hersteller von elektrochirurgischen Instrumenten vor dem Inverkehrbringen des Instruments geprüft.

Probleme können jedoch auftreten, wenn ein elektrochirurgisches Instrument nicht von einem vertrauenswürdigen Hersteller stammt. Beispielsweise kommt es immer wieder dazu, dass neuartige elektrochirurgische Instrumente von Drittherstellern nachgebaut und als Produktfälschung in Verkehr gebracht werden. Solche gefälschten Instrumente können mit charakterisierenden Daten versehen sein, die Betriebsdaten vorgeben, für welche das Instrument ggf. aufgrund unzureichender Fertigungsqualität überhaupt nicht geeignet ist. Bei der Verwendung eines solchen gefälschten elektrochirurgischen Instruments in einem elektrochirurgischen System kann es zu unzureichenden Behandlungsergebnissen oder gar zu Gefährdungen eines Patienten oder eines behandelnden Arztes kommen.

Es besteht daher eine Aufgabe der Erfindung darin, ein elektrochirurgisches System bereitzustellen, welches hinsichtlich der beschriebenen Problematik verbessert ist.

Diese Aufgabe wird gemäß eines ersten Aspekts der Erfindung gelöst durch ein elektrochirurgisches System, umfassend ein elektrochirurgisches Steuergerät und wenigstens ein elektrochirurgisches Instrument, welches mit dem elektrochirurgischen Steuergerät verbindbar ist, wobei: das wenigstens eine elektrochirurgische Instrument ein erstes Speicherelement umfasst, auf welchem das elektrochirurgische Instrument charakterisierende Daten hinterlegt oder hinterlegbar sind; das wenigstens eine elektrochirurgische Instrument eingerichtet ist, nach Verbindung mit dem elektrochirurgischen Steuergerät die das elektrochirurgische Instrument charakterisierenden Daten zumindest teilweise als erste Daten an das elektrochirurgische Steuergerät zu übermitteln; und das elektrochirurgische Steuergerät eingerichtet ist, auf Basis der von dem elektrochirurgischen Instrument übermittelten ersten Daten zulässige Betriebsparameter des elektrochirurgischen Instruments festzulegen; welches dadurch weitergebildet ist, dass auf dem ersten Speicherelement oder einem zweiten Speicherelement des elektrochirurgischen Instruments das elektrochirurgische Instrument authentifizierende Daten hinterlegt oder hinterlegbar sind; das elektrochirurgische Steuergerät eingerichtet ist, nach Verbindung mit dem elektrochirurgischen Instrument zweite Daten an das elektrochirurgische Instrument zu übermitteln; das wenigstens eine elektrochirurgische Instrument eingerichtet ist, aus den von dem elektrochirurgischen Steuergerät übermittelten zweiten Daten und den das elektrochirurgische Instrument authentifizierenden Daten abgeleitete dritte Daten an das elektrochirurgische Steuergerät zu übermitteln; und das elektrochirurgische Steuergerät eingerichtet ist, anhand der von dem elektrochirurgischen Instrument übermittelten dritten Daten einen zulässigen Funktionsumfang für das elektrochirurgische Instrument festzulegen.

Durch diese Weiterbildung kann das elektrochirurgische Steuergerät erkennen, ob das elektrochirurgische Instrument von einem vertrauenswürdigen Hersteller stammt. Dabei werden die das elektrochirurgische Instrument authentifizierenden Daten nicht direkt an das elektrochirurgische Steuergerät übertragen, so dass diese Daten nicht ohne Weiteres kopiert und für gefälschte elektrochirurgische Instrumente verwendet werden können.

Wenn das elektrochirurgische Steuergerät anhand der dritten Daten erkennt, dass es sich um ein authentisches elektrochirurgisches Instrument handelt, so kann dieses mit den anhand der ersten Daten ermittelten Betriebsparametern betrieben werden. Handelt es sich hingegen nicht um ein authentisches elektrochirurgisches Instrument, so kann der Betrieb auf einige einfache Funktionen eingeschränkt werden. Ebenso kann das elektrochirurgische Steuergerät eingerichtet sein, ein nicht authentisches elektrochirurgisches Instrument komplett abzuweisen, also überhaupt keine Funktionen zuzulassen.

In einer bevorzugten Weiterbildung eines elektrochirurgisches System gemäß der Erfindung kann das wenigstens eine elektrochirurgische Instrument eingerichtet sein, die dritten Daten nach einem kryptografischen Verfahren aus den zweiten Daten und den das elektrochirurgische Instrument authentifizierenden Daten zu ermitteln.

Unter einem kryptografischen Verfahren wird im Sinne der Erfindung ein Verfahren verstanden, welches nicht oder nur mit großem Aufwand umkehrbar ist. Es wird somit erschwert, die authentifizierenden Daten zu ermitteln, auch wenn die zweiten Daten und die dritten Daten bekannt sind, beispielsweise durch Belauschen der Kommunikation zwischen dem elektrochirurgischen Steuergerät und dem elektrochirurgischen Instrument.

Besonders bevorzugt kann das elektrochirurgische Steuergerät eingerichtet sein, die zweiten Daten zumindest teilweise nach einem Zufallsverfahren zu ermitteln. Dadurch wird es erschwert, durch Belauschen der Kommunikation zwischen dem elektrochirurgischen Steuergerät und dem elektrochirurgischen Instrument eine Liste mit zweiten Daten und zugehörigen zulässigen dritten Daten zu erstellen.

In einer möglichen Ausführung eines elektrochirurgischen Systems gemäß der Erfindung kann auf dem ersten oder dem zweiten Speicherelement ein kryptografischer Schlüssel abgelegt sein. Der kryptografische Schlüssel kann Bestandteil der das elektrochirurgische Instrument authentifizierenden Daten sein.

In einer Weiterbildung eines elektrochirurgischen Systems gemäß der Erfindung kann das elektrochirurgische Steuergerät ein drittes Speicherelement umfassen, auf welchem eine Kopie des kryptografischen Schlüssels abgelegt ist.

In einer anderen Weiterbildung eines elektrochirurgischen Systems gemäß der Erfindung kann der kryptografische Schlüssel Teil eines kryptografischen Schlüsselpaares sein, und das elektrochirurgische Steuergerät kann ein drittes Speicherelement umfassen, auf welchem der andere Teil des kryptografischen Schlüsselpaares abgelegt ist.

Entsprechend kann das kryptografische Verfahren ein symmetrisches oder ein unsymmetrisches Verfahren sein.

In einer vorteilhaften Ausführung eines elektrochirurgischen Systems gemäß der Erfindung kann das System mehrere elektrochirurgische Instrumente umfassen, welche jeweils ein erstes Speicherelement mit einem darauf abgelegten kryprografischen Schlüssel umfassen, und in dem dritten Speicherelement kann eine Kopie von jedem der kryptografischen Schlüssel bzw. zu jedem kryptografischen Schlüssel der andere Teil des entsprechenden kryptografischen Schlüsselpaares abgelegt sein.

Auf diese Weise kann ein elektrochirurgisches Steuergerät mit verschiedenen elektrochirurgischen Instrumenten betrieben werden.

Die Aufgabe wird gemäß eines zweiten Aspekts der Erfindung gelöst durch ein elektrochirurgisches Steuergerät eines elektrochirurgischen Systems gemäß den obigen Ausführungen.

Gemäß eines dritten Aspekts der Erfindung wird die Aufgabe gelöst durch ein elektrochirurgisches Instrument eines elektrochirurgischen Systems gemäß den obigen Ausführungen.

Bezüglich der dadurch erreichbaren Vorteile und Wirkungen wird jeweils auf das oben gesagte explizit verwiesen.

Die Aufgabe wird gemäß eines vierten Aspekts der Erfindung gelöst durch ein Verfahren zum Betrieb eines elektrochirurgischen Systems, umfassend ein elektrochirurgisches Steuergerät und wenigstens ein elektrochirurgisches Instrument, mit den Schritten: Verbinden des elektrochirurgischen Steuergeräts mit dem wenigstens einen elektrochirurgischen Instrument; Übermitteln von das elektrochirurgische Instrument charakterisierenden ersten Daten an das elektrochirurgische Steuergerät; und Festlegen von zulässigen Betriebsparametern für das elektrochirurgischen Instruments auf Basis der ersten Daten; Übermitteln von zweiten Daten von dem elektrochirurgischen Steuergerät an das elektrochirurgische Instrument; Ableiten von dritten Daten aus den zweiten Daten und auf dem elektrochirurgischen Instrument gespeicherten, das elektrochirurgische Instrument authentifizierenden Daten; Übermitteln der dritten Daten an das elektrochirurgische Steuergerät; und Festlegen eines für das elektrochirurgische Instrument zulässigen Funktionsumfangs durch das elektrochirurgische Steuergerät anhand der dritten Daten.

Dabei können die dritten Daten vorzugsweise nach einem kryptografischen Verfahren aus den zweiten Daten und den das elektrochirurgische Instrument authentifizierenden Daten abgeleitet werden.

Die zweiten Daten können besonders bevorzugt nach einem Zufallsverfahren ermittelt werden.

Die Erfindung wird nachfolgend anhand einer exemplarischer Zeichnungen näher erläutert. Dabei sollen die im Folgenden dargestellten Ausführungsbeispiele lediglich zum besseren Verständnis der Erfindung beitragen, ohne diese einzuschränken.

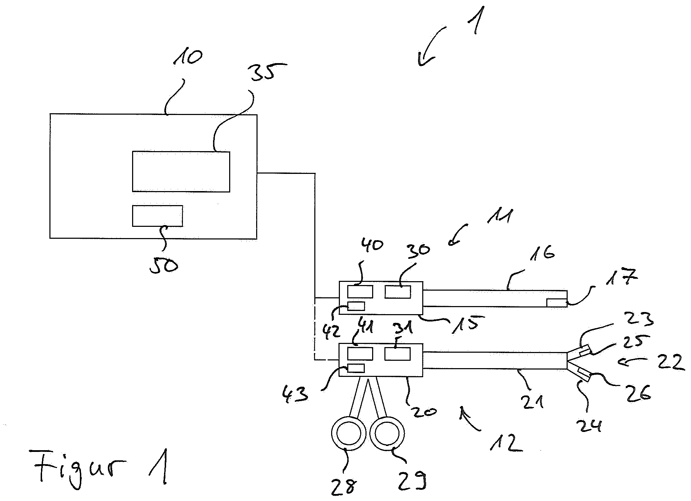

Die Figur 1 zeigt ein elektrochirurgisches System.

In Figur 1 ist ein elektrochirurgisches System 1 dargestellt. Das elektrochirurgische System 1 umfasst ein elektrochirurgisches Steuergerät 10, bei welchem es sich im dargestellten Beispiel um einen elektrochirurgischen Generator handelt. Weiterhin umfasst das elektrochirurgische System 1 zwei elektrochirurgische Instrumente 11, 12, die wechselweise oder gleichzeitig an das elektrochirurgische Steuergerät 10 angeschlossen werden können.

Das elektrochirurgische Instrument 11 umfasst einen Hauptkörper 15, der als Griffstück dienen kann, und einen langgestreckten Schaft 16, an dessen distalem Ende eine Elektrode 17 angeordnet ist. Bei dem elektrochirurgischen Instrument 11 kann es sich beispielsweise um ein monopolares elektrochirurgisches Skalpell handeln.

Das elektrochirurgische Instrument 12 umfasst ebenfalls einen Hauptkörper 20 und einen langgestreckten Schaft 21. Anders als das elektrochirurgische Instrument 11 weist das elektrochirurgische Instrument 12 ein Zangenmaul 22 mit beweglichen Branchen 23, 24 auf. Die Branchen 23, 24 tragen jeweils eine Behandlungselektrode 25, 26. An dem Hauptkörper 20 sind weiterhin zwei Griffhebel 28, 29 angeordnet, welche zum Betätigen der Branchen 23, 24 relativ zueinander beweglich sind. Bei dem elektrochirurgischen Instrument 12 kann es sich beispielsweise um eine bipolare Koagulationszange handeln.

Die elektrochirurgischen Instrumente 11, 12 sind jeweils mit einem ersten Speicherelement 30, 31 ausgerüstet, welches nach dem Anschließen der elektrochirurgischen Instrumente 11, 12 an das elektrochirurgische Steuergerät 10 von diesem ausgelesen werden können. Dazu umfasst das elektrochirurgische Steuergerät 10 eine Steuerung 35. Auf den ersten Speicherelementen 30, 31 sind Daten hinterlegt, welche die elektrochirurgischen Instrumente 11, 12 charakterisieren. Diese Daten können beispielsweise Typkennungen der elektrochirurgischen Instrumente 11, 12 umfassen, und/der direkt zulässige Parameter eines von dem elektrochirurgischen Steuergerät 10 an die elektrochirurgischen Instrumente 11, 12 abzugebenden Wechselstroms.

Auf Basis der von den ersten Speicherelementen 30, 31 gelesenen Daten legt die Steuerung 35 die Betriebsparameter fest, mit welchen die elektrochirurgischen Instrumente 11, 12 betrieben werden können. Das elektrochirurgische Steuergerät 10 kann eine nicht dargestellte Benutzerschnittstelle umfassen, über welche diese Betriebsparameter durch einen Benutzer des elektrochirurgischen Systems 1 weiter angepasst werden können. Beispielsweise kann die Steuerung 35 auf Basis der von den ersten Speicherelementen 30, 31 gelesenen Daten einen Rahmen festlegen, innerhalb dessen die Betriebsparameter von einem Benutzer über die Benutzerschnittstelle frei gewählt werden können.

Um sicherzustellen, dass die elektrochirurgischen Instrumente 11, 12 aus einer vertrauenswürdigen Quelle stammen, weisen diese zusätzlich zweite Speicherelemente 40, 41 auf, auf welchen Daten hinterlegt sind, mit denen die elektrochirurgischen Instrumente 11, 12 authentifiziert werden können. Bei den auf den Speicherelementen 40, 41 gespeicherten Authentifizierungsdaten kann es sich um kryptografische Schlüssel handeln.

Um eine direkte Übertragung der Authentifikationsdaten zu vermeiden, die prinzipiell abgehört werden könnte, ist den zweiten Speicherelementen 40, 41 jeweils ein Controller 42, 43 zugeordnet. Die Controller 42, 43 sind eingerichtet, Anfragedaten zu empfangen, diese über eine kryptografische Funktion mit auf dem Speicherelement 40 bzw. 41 gespeicherten Authentifizierungsdaten zu verknüpfen, und das Ergebnis der Verknüpfung als Antwortdaten auszugeben.

Im Folgenden sind zwei mögliche Authentifizierungsverfahren für die elektrochirurgischen Instrumente 11, 12 dargestellt.

Ein erstes Authentifizierungsverfahren beruht auf einem symmetrischen Verschlüsselungsverfahren. Dabei ist auf dem zweiten Speicherelement 40 des elektrochirurgischen Instruments 11 ein kryptografischer Schlüssel K1 gespeichert, und eine Kopie des Schlüssels K1 ist in einem dritten Speicherelement 50 des elektrochirurgischen Steuergeräts 10 hinterlegt.

Wenn nun das elektrochirurgische Instrument 11 mit dem elektrochirurgischen Steuergerät 10 verbunden wird, so erzeugt die Steuerung 35 zunächst eine Zufallszahl Z und sendet diese an das elektrochirurgische Instrument 11. In dem elektrochirurgischen Instrument 11 empfängt der Controller 42 die Zufallszahl Z und verschlüsselt diese mit dem auf dem zweiten Speicherelement 40 gespeicherten Schlüssel K1 zu einem Rückgabewert R=f(Z,K1). Der Rückgabewert R wird an die Steuerung 35 zurück übermittelt.

Die Steuerung 35 liest nun die Kopie des Schlüssels K1 aus dem dritten Speicherelement 50 und entschlüsselt mit diesem den Rückgabewert R nach der Umkehrfunktion f⁻¹. Wenn das Ergebnis der Entschlüsselung Z'=f⁻¹(R,K1) dem ursprünglichen Zufallswert Z entspricht, so ist das elektrochirurgische Instrument 11 erfolgreich authentifiziert. Andernfalls handelt es sich bei dem elektrochirurgischen Instrument 11 möglicherweise um eine Fälschung. Im letzteren Fall kann die Steuerung 35 einen möglichen Funktionsumfang für das elektrochirurgische Instrument 11 einschränken, um eine Gefährdung den Patienten oder des Benutzers des elektrochirurgischen Systems 1 auszuschließen. Die Steuerung 35 kann auch einen Betrieb des elektrochirurgischen Instruments 11 komplett unterbinden.

In dem zweiten Speicherelement 41 des elektrochirurgischen Instruments 12 kann ein zweiter Schlüssel K2 gespeichert sein, eine Kopie des zweiten Schlüssels K2 ist ebenfalls in dem dritten Speicherelement 50 hinterlegt. Das elektrochirurgische Steuergerät 10 kann somit mehrere Arten von elektrochirurgischen Instrumenten 11, 12 authentifizieren.

Verfügbare symmetrische Verschlüsselungsverfahren sind z.B. "AES", "Blowfish" oder "Twofish".

Ein zweites mögliches Authentifizierungsverfahren beruht auf einem asymmetrischen Verschlüsselungsverfahren. Hierbei ist auf dem zweiten Speicherelement 40 des elektrochirurgischen Instruments 11 ein privater Schlüssel P1 hinterlegt, auf dem dritten Speicherelement 50 hingegen ein dazugehöriger öffentlicher Schlüssel Ö1.

Um das elektrochirurgische Instrument 11 zu authentifizieren, erzeugt die Steuerung 35 zunächst wieder eine Zufallszahl Z, verschlüsselt diese nach der Funktion A=g(Z,Ö1), um den Anfragewert A zu erhalten, und sendet den Anfragewert A an das elektrochirurgische Instrument 11.

Der Controller 42 empfängt den Anfragewert A und entschlüsselt ihn unter Nutzung des privaten Schlüssels P1 nach der Funktion R=g'(A,P1), um den Rückgabewert R zu erhalten, und sendet den Rückgabewert R an das elektrochirurgische Steuergerät 10 zurück.

Hier prüft die Steuerung 35 nun, ob der Rückgabewert R der Zufallszahl Z entspricht. In diesem Fall ist das elektrochirurgische Instrument 11 erfolgreich authentifiziert, andernfalls erfolgt wie oben beschrieben eine teilweise oder vollständige Einschränkung des zur Verfügung gestellten Funktionsumfangs.

Für mehrere Arten von elektrochirurgische Instrumenten 11, 12 können ähnlich zu den obigen Ausführungen mehrere Schlüsselpaare Ö1,P1, Ö2,P2 verwendet werden.

Die Verwendung eines asymmetrischen Verschlüsselungsverfahrens hat den Vorteil, dass bei Einführung neu entwickelter elektrochirurgischer Instrumente, für die ein neuer kryptografischen Schlüssel genutzt werden soll, der dazugehörende öffentliche Schlüssel unkompliziert an im Feld befindliche elektrochirurgische Steuergeräte verteilt werden kann, ohne dass hierzu der private Schlüssel offenbart werden muss. Bei Verwendung eines symmetrischen Verschlüsselungsverfahrens muss bei der Verteilung des neuen Schlüssels an im Feld befindliche elektrochirurgische Steuergeräte sichergestellt sein, dass der Schlüssel hierbei nicht in falsche Hände gerät.

Mögliche asymmetrische Verschlüsselungsverfahren sind beispielsweise "RSA" oder das "Elgamal"-Verfahren.

Um einen Zugriff auf den Speicherinhalt der zweiten Speicherelemente 40, 41 zu verhindern, sind diese mit den zugehörigen Controllern 42 bzw. 43 zu einem Sicherheitsbaustein zusammengefasst. Dies kann beispielsweise ein "Trusted Platform Module" (TPM) nach der Spezifikation der "Trusted Computing Group" (TCG) sein.

Um den Aufbau der elektrochirurgischen Instrumente 11, 12 zu vereinfachen, können jeweils die ersten und zweiten Speicherelemente 30 und 40 bzw. 31 und 41 zu einem einzigen Speicherelement zusammengefasst sein.

## Patentansprüche

1. Elektrochirurgisches System, umfassend
- ein elektrochirurgisches Steuergerät (10) und
- wenigstens ein elektrochirurgisches Instrument (11, 12), welches mit dem elektrochirurgischen Steuergerät (10) verbindbar ist, wobei:
- das wenigstens eine elektrochirurgische Instrument (11, 12) ein erstes Speicherelement (30, 31) umfasst, auf welchem das elektrochirurgische Instrument (11, 12) charakterisierende Daten hinterlegt oder hinterlegbar sind;
- das wenigstens eine elektrochirurgische Instrument (11, 12) eingerichtet ist, nach Verbindung mit dem elektrochirurgischen Steuergerät (10) die das elektrochirurgische Instrument (11, 12) charakterisierenden Daten zumindest teilweise als erste Daten an das elektrochirurgische Steuergerät (10) zu übermitteln; und
- das elektrochirurgische Steuergerät (10) eingerichtet ist, auf Basis der von dem elektrochirurgischen Instrument (11, 12) übermittelten ersten Daten zulässige Betriebsparameter des elektrochirurgischen Instruments (11, 12) festzulegen; **dadurch gekennzeichnet, dass**
- auf dem ersten Speicherelement (30, 31) oder einem zweiten Speicherelement (40, 41) des elektrochirurgischen Instruments (11, 12) das elektrochirurgische Instrument (11, 12) authentifizierende Daten hinterlegt oder hinterlegbar sind,
- das elektrochirurgische Steuergerät (10) eingerichtet ist, nach Verbindung mit dem elektrochirurgischen Instrument (11, 12) zweite Daten an das elektrochirurgische Instrument (11, 12) zu übermitteln,
- das wenigstens eine elektrochirurgische Instrument (11, 12) eingerichtet ist, aus den von dem elektrochirurgischen Steuergerät (10) übermittelten zweiten Daten und den das elektrochirurgische Instrument (11, 12) authentifizierenden Daten abgeleitete dritte Daten an das elektrochirurgische Steuergerät (10) zu übermitteln, und
- das elektrochirurgische Steuergerät (10) eingerichtet ist, anhand der von dem elektrochirurgischen Instrument (11, 12) übermittelten dritten Daten einen zulässigen Funktionsumfang für das elektrochirurgische Instrument (11, 12) festzulegen.

2. Elektrochirurgisches System nach Anspruch 1, **dadurch gekennzeichnet, dass** das wenigstens eine elektrochirurgische Instrument (11, 12) eingerichtet ist, die dritten Daten nach einem kryptografischen Verfahren aus den zweiten Daten und den das elektrochirurgische Instrument (11, 12) authentifizierenden Daten zu ermitteln.

3. Elektrochirurgisches System nach Anspruch 1 oder 2, **dadurch gekennzeichnet, dass** das elektrochirurgische Steuergerät (10) eingerichtet ist, die zweiten Daten zumindest teilweise nach einem Zufallsverfahren zu ermitteln.

4. Elektrochirurgisches System nach Anspruch 2 oder 3, **dadurch gekennzeichnet, dass** auf dem ersten und/oder zweiten Speicherelement (30, 31, 40, 41) ein kryptografischer Schlüssel abgelegt ist.

5. Elektrochirurgisches System nach Anspruch 4, **dadurch gekennzeichnet, dass** das elektrochirurgische Steuergerät (10) ein drittes Speicherelement (50) umfasst, auf welchem eine Kopie des kryptografischen Schlüssels abgelegt ist.

6. Elektrochirurgisches Instrument nach Anspruch 4, **dadurch gekennzeichnet, dass** der kryptografische Schlüssel Teil eines kryptografischen Schlüsselpaares ist, und dass das elektrochirurgische Steuergerät (10) ein drittes Speicherelement (50) umfasst, auf welchem der andere Teil des kryptografischen Schlüsselpaares abgelegt ist.

7. Elektrochirurgisches System nach Anspruch 5 oder 6, wobei das System mehrere elektrochirurgische Instrumente (11, 12) umfasst, welche jeweils ein erstes oder zweites Speicherelement (30, 31, 40, 41) mit einem darauf abgelegten kryprografischen Schlüssel umfassen, und dass in dem dritten Speicherelement (50) eine Kopie von jedem der kryptografischen Schlüssel bzw. zu jedem kryptografischen Schlüssel der andere Teil des entsprechenden kryptografischen Schlüsselpaares abgelegt ist.

8. Elektrochirurgisches Steuergerät (10) eines elektrochirurgischen Systems nach einem der Ansprüche 1 bis 7.

9. Elektrochirurgisches Instrument (11, 12) eines elektrochirurgischen Systems nach einem der Ansprüche 1 bis 7.

10. Verfahren zum Betrieb eines elektrochirurgischen Systems, umfassend ein elektrochirurgisches Steuergerät (10) und wenigstens ein elektrochirurgisches Instrument (11, 12), mit den Schritten:
- Verbinden des elektrochirurgischen Steuergeräts (10) mit dem wenigstens einen elektrochirurgischen Instrument (11, 12),
- Übermitteln von das elektrochirurgische Instrument (11, 12) charakterisierenden ersten Daten an das elektrochirurgische Steuergerät (10), und
- Festlegen von zulässigen Betriebsparametern für das elektrochirurgische Instrument (11, 12) auf Basis der ersten Daten,
- Übermitteln von zweiten Daten von dem elektrochirurgischen Steuergerät (10) an das elektrochirurgische Instrument (11, 12),
- Ableiten von dritten Daten aus den zweiten Daten und auf dem elektrochirurgischen Instrument (11, 12) gespeicherten, das elektrochirurgische Instrument (11, 12) authentifizierenden Daten,
- Übermitteln der dritten Daten an das elektrochirurgische Steuergerät (10), und
- Festlegen eines für das elektrochirurgische Instrument (11, 12) zulässigen Funktionsumfangs durch das elektrochirurgische Steuergerät (10) anhand der dritten Daten.

11. Verfahren nach Anspruch 10, wobei die dritten Daten nach einem kryptografischen Verfahren aus den zweiten Daten und den das elektrochirurgische Instrument (11, 12) authentifizierenden Daten abgeleitet werden.

12. Verfahren nach Anspruch 11, **dadurch gekennzeichnet, dass** die zweiten Daten nach einem Zufallsverfahren ermittelt werden.
